# EUROPEAN PATENT SPECIFICATION

(11) **EP 4 593 951 B1**
(45) Date of publication and mention of the grant of the patent: **28.10.2026**
(21) Application number: 23776588.8
(22) Date of filing: 19.09.2023
(51) Int. Cl.: A61N 5/10, G16H 40/67, G16H 50/20, G16H 50/50, G16H 50/70, G06N 20/00, G16H 20/40

(54) **RADIATION THERAPY PLAN GENERATION USING AUTOMATED TREATABLE SECTORS**
STRAHLENTHERAPIEPLANERZEUGUNG MIT AUTOMATISIERTEN BEHANDELBAREN SEKTOREN
GÉNÉRATION DE PLAN DE RADIOTHÉRAPIE EN UTILISANT DES SECTEURS POUVANT ÊTRE TRAITÉS DE MANIÈRE AUTOMATISÉS

(30) Priority: 30.09.2022 US 202217958225
(43) Date of publication of application: 06.08.2025
(73) Proprietor: Siemens Healthineers International AG, 6312 Steinhausen (CH)
(72) Inventor: HYVONEN, Heini, 00560 Helsinki (FI); RUOKOKOSKI, Emmi, 00380 Helsinki, Uusimaa (FI)
(74) Representative: Mathisen & Macara LLP
(86) International application number: PCT/EP2023/075845
(87) International publication number: WO 2024/068371

(56) References cited:
- US-A1- 2019 074 079
- US-A1- 2022 161 062
- JIA QIYUAN ET AL: "OAR Dose Distribution Prediction and gEUD Based Automatic Treatment Planning Optimization for Intensity Modulated Radiotherapy", IEEE ACCESS, vol. 7, 19 September 2019 (2019-09-19), pages 141426 - 141437, XP011749507, DOI: 10.1109/ACCESS.2019.2942393

## Description

### TECHNICAL FIELD

This application relates generally to using computer modeling techniques to generate radiation therapy treatment plans to control a radiation therapy machine.

### BACKGROUND

Radiation therapy (radiation-based therapy or radiotherapy) is used as a cancer treatment to emit high doses of radiation that can kill cells or shrink a tumor. The target region of a patient's body that is intended to receive radiation (e.g., tumor) is referred to as the planning target volume (PTV). The goal is to deliver enough radiation to the PTV to kill the cancerous cells during a radiation therapy treatment (also referred to herein as a treatment plan or radiation therapy treatment). However, other organs or anatomical regions that are adjacent to or surround the PTV can be in the path of radiation beams and can receive enough radiation to damage or harm such organs or anatomical regions. These organs or anatomical regions are referred to as organs at risk (OARs). Usually, a physician or a radiologist identifies both the PTV and the OARs before radiation therapy using, for example, computed tomography (CT) images, magnetic resonance imaging (MRI) images, positron emission tomography (PET) images, images obtained via some other imaging modality, or a combination thereof. For example, US 2022/161062 Al discloses method comprising:
- receiving, by a processor, radiation therapy treatment planning data associated with a radiation therapy treatment of a patient;
- executing, by the processor, a first computer model to identify one or more attributes of a treatable sector for the radiation therapy treatment of the patient, the first computer model configured to ingest the radiation therapy treatment planning data to generate the one or more attributes of the treatable sector; and
- identification of a radiation therapy treatment plan for the patient using the radiation therapy treatment planning data associated with the patient, using the one or more attributes of the treatable sector identified by the first computer model.

Using the medical images of the patient as well as the identified PTV and the OARs, a team of medical professionals (e.g., physicians, radiologists, oncologists, radiology technicians, other medical personnel, or a combination thereof) determines the radiation parameters to be used during the radiation therapy treatment. These radiation parameters may include, for example, the type, the angle, the radiation intensity, and/or the shape of each radiation beam. In determining these parameters, the medical professionals attempt to achieve a radiation dose distribution to deliver to the patient that meets predefined criteria (also referred to herein as the plan objectives or clinical objectives). Such criteria usually include predefined radiation dose thresholds or ranges for the PTV and the OARs.

To optimize the radiation parameters in a way to meet the predefined criteria, software solution(s) or a computer model(s) (typically referred to as "plan optimizers") are utilized that can run a plurality of simulations with various radiation parameters and can select a final set of radiation parameters to be used based on the simulation results. These radiation parameters are typically referred to as a radiation therapy treatment plan (RTTP). However, this process can be highly inefficient and undesirable because plan optimizers typically use an iterative, trial-and-error process to optimize various attributes of the RTTP. For instance, after the treating physician inputs the objectives, the software solution iteratively analyzes different possibilities (e.g., different iterations of different attributes within a large search space) to identify one or more beam geometry attributes that yield the best/acceptable results. As a result, the software solutions may require substantial computing resources and may not produce timely results.

Conventional solutions suffer from technical shortcomings because defining beam geometry for treatment planning is especially a complicated process where target size, location, dose prescription, patient condition, and treatment machine limitations (including the risk of collision) all play a role. Designing an automatic algorithm to generate the RTTP is technically challenging because existing solutions provide a mathematical approach that does not consider contextual information regarding the treatment itself. As a result, conventional solutions may place beams/arcs through clinically unacceptable regions simply because the dose distribution satisfies the provided clinical goal limits and minimizes the objective function defined in a mathematical sense. An example of such a situation arises, for example, when a beam enters through the shoulder, nerve roots, or healthy lung. Sometimes, for example, a lung tumor may be centrally located and the beam geometry optimization algorithm may consider both lungs as equally important due to the clinical protocol. Hence, the beam geometry optimization algorithm may place beam/arcs to enter through both lungs. However, the clinical best practice is to avoid placing beam entries through the contra lung. In this example, the beam is mathematically optimized but not medically acceptable. As a result, the algorithm must restart the iteration process to revise the RTTP, which is time-consuming and computationally inefficient.

### SUMMARY

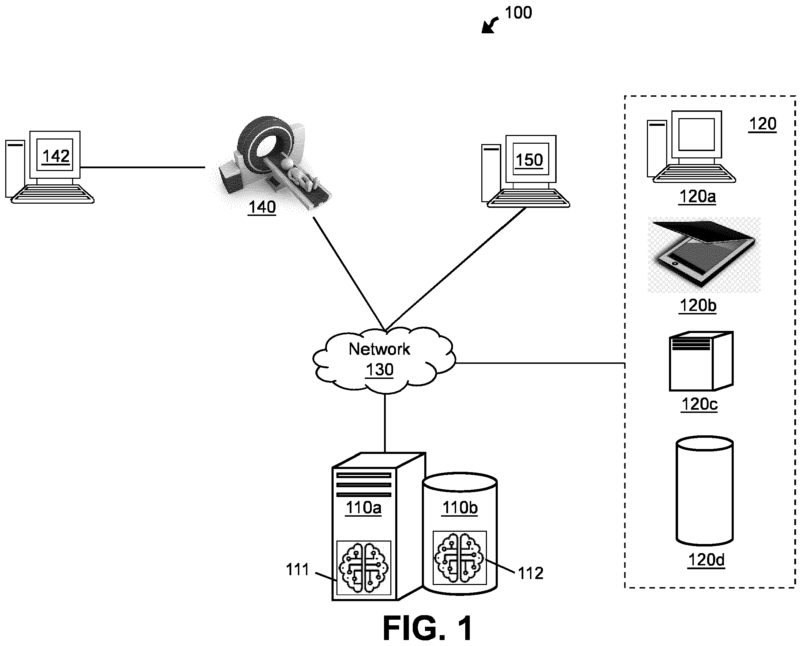

For the aforementioned reasons, there is a desire for a system that can rapidly and accurately analyze plan/treatment objectives and patient information to provide RTTP attributes. Using the methods and systems discussed herein, a processor (e.g., the analytics server discussed in FIG. 1) can define treatable sectors based on anatomical site, laterality, and tumor location(s) compared to OARs and body outlines. Moreover, using the treatable sector, the analytics server may limit the search space used by the plan optimizer, such that efficiencies are created. For instance, the plan optimizer may provide an RTTP (e.g., including beam geometry) that complies with various criteria (e.g., best practices) faster, without requiring user intervention, and/or uses less computing resources.

Conventional methods may produce results that violate clinical best practices while not violating the mathematical formula with which they optimize the RTTP. Using the methods and systems discussed herein, a server/computer may use the clinical site, laterality, and tumor location, and other pertinent information to define a treatable sector. The treatable sector can then be ingested by a plan optimizer, such that the plan optimizer can become more efficient. Using the methods and systems discussed herein, the RTTP can be optimized without requiring user intervention, such as user inputs regarding adjusting beam geometries. Unlike some other approaches, such as those using deep learning or other artificial intelligence approaches, the methods and systems discussed herein do not require extensive model training. Thereby, implementing the methods and system discussed herein may be faster and simpler and can be added to existing plan optimizers and existing RTTP generation system architectures.

In one aspect, the present invention provides a method as defined in claim 1. Optional features are specified in the dependent claims.

In another aspect, the present invention provides a system as defined in claim 8. Optional features are specified in the dependent claims.

In another aspect, the present invention provides a system as defined in claim 15. Optional features are specified in the dependent claims.

The treatable sector may correspond to a plurality of ranges of beam entry angles. The search space used by the second computer model may be limited to a subset of the plurality of ranges of beam entry angles.

In an embodiment, a method comprises receiving, by a processor, radiation therapy treatment planning data associated with a radiation therapy treatment of a patient; executing, by the processor, a first computer model to identify one or more attributes of a treatable sector for the radiation therapy treatment of the patient, the first computer model configured to ingest radiation therapy treatment planning data and clinical objectives to generate the one or more attributes of the treatable sector; and executing, by the processor, a second computer model to identify a radiation therapy treatment plan for the patient using the received radiation therapy treatment planning data associated with a patient, wherein the processor limits a search space used by the second computer model using the one or more attributes of the treatable sector identified by the first computer model.

The first computer model may use a machine-learning algorithm to predict the one or more attributes of the treatable sector.

The machine-learning algorithm may train the first computer model using training data comprising data associated with previously implemented treatments.

The one or more attributes of the treatable sector may be calculated based on tumor location.

The one or more attributes of the treatable sector may be calculated based on data associated with an organ at risk.

The one or more attributes of the treatable sector may correspond to a range of angles associated with beam entry.

The treatable sector may correspond to a plurality of ranges of beam entry angles..

In another embodiment, a system comprises a computer-readable medium having a set of non-transitory instructions, that when executed, cause a processor to receive radiation therapy treatment planning data associated with a radiation therapy treatment of a patient; execute a first computer model to identify one or more attributes of a treatable sector for the radiation therapy treatment of the patient, the first computer model configured to ingest (or use or process) radiation therapy treatment planning data and clinical objectives to generate the one or more attributes of the treatable sector; and execute a second computer model to identify a radiation therapy treatment plan for the patient using the received radiation therapy treatment planning data associated with a patient, wherein the processor limits a search space used by the second computer model using the one or more attributes of the treatable sector identified by the first computer model.

The first computer model may use a machine-learning algorithm to predict the one or more attributes of the treatable sector.

The machine-learning algorithm may train the first computer model using training data comprising data associated with previously implemented treatments.

The one or more attributes of the treatable sector may be calculated based on tumor location.

The one or more attributes of the treatable sector may be calculated based on data associated with an organ at risk.

The one or more attributes of the treatable sector may correspond to a range of angles associated with beam entry.

The treatable sector may correspond to a plurality of ranges of beam entry angles.

In another embodiment, a system comprises a first computer model configured to ingest radiation therapy treatment planning data and clinical objectives to generate one or more attributes of the treatable sector; and a server in communication with the first computer model, the server configured to receive radiation therapy treatment planning data associated with a radiation therapy treatment of a patient; execute the first computer model to identify one or more attributes of the treatable sector for the radiation therapy treatment of the patient; and execute a second computer model to identify a radiation therapy treatment plan for the patient using the received radiation therapy treatment planning data associated with a patient, wherein the processor limits a search space used by the second computer model using the one or more attributes of the treatable sector identified by the first computer model.

The first computer model may use a machine-learning algorithm to predict the one or more attributes of the treatable sector.

The machine-learning algorithm may train the first computer model using training data comprising data associated with previously implemented treatments.

The one or more attributes of the treatable sector may be calculated based on tumor location.

The one or more attributes of the treatable sector may be calculated based on data associated with an organ at risk.

The one or more attributes of the treatable sector may correspond to a range of angles associated with beam entry.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a plan generation system, according to an embodiment.
**FIG. 2** illustrates a process flow diagram executed in a plan generation system, according to an embodiment.
**FIG. 3** illustrates different visual representations of treatable sectors, according to an embodiment.
**FIG. 4** illustrates a visual representation of treatable sectors, according to an embodiment.
**FIG. 5** illustrates a visual representation of a non-limiting example of implementing the plan generation system, according to an embodiment.

### DETAILED DESCRIPTION

The invention is defined in the following claims.

**FIG.** 1 illustrates components of a plan generation system 100 (system 100), according to an embodiment. The system **100** may include an analytics server **110a,** system database **110b**, treatable sector model **111**, end-user devices **120a-d** (collectively end-user devices **120**), and an administrator computing device **150**. Various components depicted in **FIG. 1** may belong to a radiation therapy clinic at which patients may receive radiation therapy treatment, in some cases via one or more radiation therapy machines located within the clinic (e.g., medical device **140**). The above-mentioned components may be connected through a network **130**. Examples of the network **130** may include but are not limited to, private or public LAN, WLAN, MAN, WAN, and the Internet. The network **130** may include wired and/or wireless communications according to one or more standards and/or via one or more transport mediums.

The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), and EDGE (Enhanced Data for Global Evolution) network.

The system **100** is not confined to the components described herein and may include additional or other components, not shown for brevity, which is to be considered within the scope of the embodiments described herein.

The analytics server **110a** may generate and display an electronic platform configured to use various computer models **111-112** to identify attributes of an RTTP for a patient's treatment. The electronic platform may include a graphical user interface (GUI) displayed on the end-user devices **120** and/or the administrator computing device **150.** An example of the electronic platform generated and hosted by the analytics server **110a** may be a web-based application or a website configured to be displayed on different electronic devices, such as mobile devices, tablets, personal computers, and the like.

In a non-limiting example, a physician operating the physician device **120b** may access the platform, input patient attributes or characteristics and other data, and further instruct the analytics server **110a** to generate an RTTP (including the beam angle). Additionally or alternatively, the analytics server **110a** may only optimize a particular attribute of the RTTP (e.g., optimize beam angle only).

The analytics server **110a** may recommend the RTTP (e.g., beam angles and other radiation parameters and/or treatment plan attributes) used for proton radiation, photon radiation, and electron radiation. In particular, analytics server **110a** may utilize the methods and systems described herein to execute the treatable sector model **111** to identify treatable sectors. Then, the analytics server **110a** may use the generated treatable sectors to limit the search space used by the plan optimizer model **112**. Further, the analytics server **110a** may transmit the beam angles and other radiation parameters and/or treatment plan attributes to one or more other servers. Additionally, or alternatively, the analytics server **110a** (or another server) may adjust the configuration of one or more devices (e.g., the medical device **140**) based on the optimized beam angle. For instance, the RTTP may be directly sent to the medical device **140** and/or the computer **142** that functionally controls the medical device **140**.

The medical device **140** may be any medical device used in the radiation therapy treatment of a patient (such as a CT scan machine, radiation therapy machine (e.g., a linear accelerator, particle accelerator (including circular accelerators), or a cobalt machine)). The medical device **140** may also include an imaging device capable of emitting radiation such that the medical device **140** may perform imaging according to various methods to accurately image the internal structure of a patient. For instance, the medical device **140** may include a rotating system (e.g., a static or rotating multi-view system). A non-limiting example of a multi-view system may include stereo systems (e.g., two systems may be arranged orthogonally).

The analytics server **110a** may host a website accessible to users operating any of the electronic devices described herein (e.g., end users or medical professionals), where the content presented via the various webpages may be controlled based upon each particular user's role or viewing permissions. The analytics server **110a** may be any computing device comprising a processor and non-transitory machine-readable storage capable of executing the various tasks and processes described herein. The analytics server **110a** may employ various processors such as central processing units (CPU) and graphics processing unit (GPU), among others. Non-limiting examples of such computing devices may include workstation computers, laptop computers, server computers, and the like. While the system **100** includes a single analytics server **110a,** the analytics server **110a** may include any number of computing devices operating in a distributed computing environment, such as a cloud environment.

The analytics server **110a** may execute software applications configured to display the electronic platform (e.g., host a website), which may generate and serve various webpages to each end-user devices **120.** Different users may use the website to view and/or interact with the recommended (optimized) results. Different servers, such as a clinic server **120c** may also use the recommended results in downstream processing.

The analytics server **110a** may be configured to require user authentication based upon a set of user authorization credentials (e.g., username, password, biometrics, cryptographic certificate, and the like). The analytics server **110a** may access the system database **110b** configured to store user credentials, which the analytics server **110a** may be configured to reference in order to determine whether a set of entered credentials (purportedly authenticating the user) match an appropriate set of credentials that identify and authenticate the user.

The analytics server **110a** may generate and host webpages based upon a particular user's role within the system **100.** In such implementations, the user's role may be defined by data fields and input fields in user records stored in the system database **110b.** The analytics server **110a** may authenticate the user and may identify the user's role by executing an access directory protocol (e.g., LDAP). The analytics server **110a** may generate webpage content that is customized according to the user's role defined by the user record in the system database **110b.**

The analytics server **110a** may receive various clinical objectives, patient data, and treatment data from the end-user devices **120.** For instance, a physician may access the platform provided by the analytics server **110a** using a physician device **120b.** The physician may input various patient attributes and/or clinical objectives using one or more input elements of the platform. The analytics server **110a** may then execute various methods discussed herein and display an RTTP on the platform.

The end-user devices **120** may be any computing device comprising a processor and a non-transitory machine-readable storage medium capable of performing the various tasks and processes described herein. Non-limiting examples of end-user devices **120** may be a workstation computer, laptop computer, tablet computer, and server computer. In operation, various users may use end-user devices **120** to access the GUI operationally managed by the analytics server **110a.** Specifically, the end-user devices **120** may include clinic computer **120a,** physician device **120b,** clinic server **120c,** and clinic database **120d.**

In order to generate the RTTP, the analytics server **110a** may then execute various models (e.g., treatable sector model **111** and/or a plan optimizer model **112)** to analyze the retrieved/received data.

The administrator computing device **150** may represent a computing device operated by a system administrator. The administrator computing device **150** may be configured to display beam angles, radiation parameters, and/or other radiation therapy treatment attributes generated by the analytics server **110a;** monitor the treatable sector model **111** utilized by the analytics server **110a,** and/or end-user devices **120;** review feedback; and/or facilitate training or retraining (calibration) of the models **111-112** that are maintained by the analytics server **110a.**

The analytics server **110a** may be in communication (real-time or near real-time) with the medical device **140** (or its computer **142),** such that a server/computer hosting the medical device **140** can adjust the medical device **140** based on the RTTP (e.g., beam angles, treatment attributes and/or radiation parameters determined by the analytics server **110a).** For instance, the radiation therapy machine may adjust the gantry, beam blocking device (e.g. multi-leaf collimator MLC), and couch based on optimized beam angles, where the optimized beam angle is an angle of the medical device **140** that emits radiation in a direction of the PTVs. The analytics server **110a** may transmit instructions to the radiation therapy machines indicating any number or type of radiation parameters and/or treatment attributes to facilitate such adjustments.

The models **111** and/or **112** may represent any collection of algorithmic logic and/or artificial intelligence models. For instance, the treatable sector model **111** may include various algorithms to identify attributes and characteristics of a treatable sector based on patient data and/or treatment attributes. Using the treatable sector, the analytics server **110a** may execute the plan optimizer model **112.** For instance, the analytics server **110a** may execute the models in conjunction with each other and then revise one or more configuration parameters of the plan optimizer **112.** Non-limiting examples of a configuration parameter revised by the analytics server **110a** may include a search space that is limited/revised using the attributes of the treatable sector. As a result, the efficiencies discussed herein can be achieved without revising the plan optimizer **112** itself. For instance, the treatable sector model **111** can be executed independently and used in conjunction with an existing plan optimizer. Therefore, the plan optimizer (or the system infrastructure) can be retrofitted using the methods and systems discussed herein. This minimal interference with existing infrastructure allows for the improvement of a plan optimizer without the need to revise its source code.

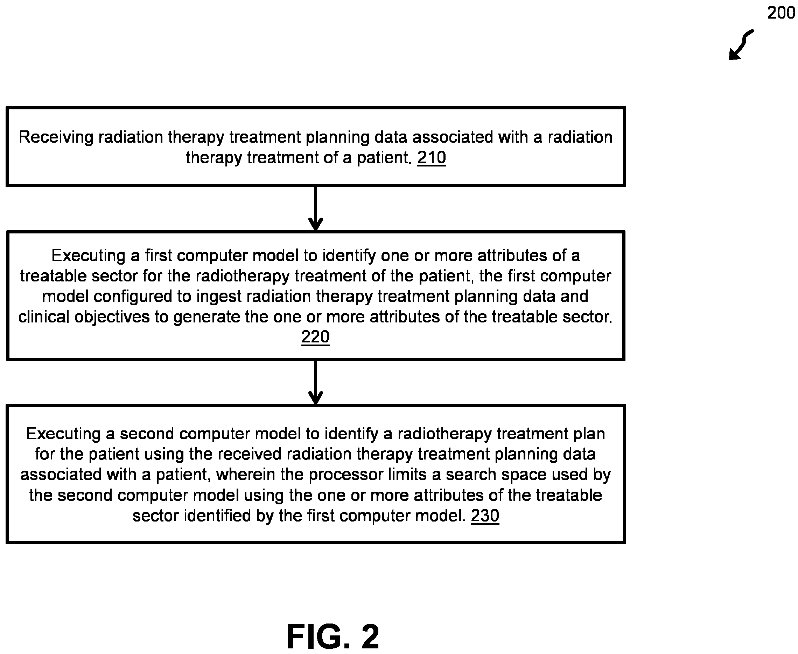

**FIG. 2** illustrates a flow diagram of a process executed in a plan generation system, according to an embodiment. The method **200** includes steps **210-230.** However, other embodiments may include additional or alternative execution steps or may omit one or more steps altogether. The method **200** is described as being executed by a server, similar to the analytics server described in **FIG. 1****.** However, one or more steps of method **200** may also be executed by any number of computing devices operating in the distributed computing system described in **FIG. 1****.** For instance, one or more user computing devices may locally perform part or all the steps described in **FIG. 2****.**

At step **210,** the analytics server may receive radiation therapy treatment planning data associated with a radiation therapy of a patient. The received radiation therapy treatment planning data may refer to radiation-therapy-specific information associated with the patient and may include patient data, clinic-specific data, and clinical objectives (e.g., treatment requirements and attributes whether they are mandatory or preferred by a treating physician). The radiation therapy treatment planning data may refer to data associated with a process in which a medical team (e.g., radiation oncologists, radiation therapists, medical physicists, and/or medical dosimetrists) plan the appropriate external beam radiation therapy or internal brachytherapy treatment techniques for the patient. In some configurations, the analytics server may receive the data associated with a patient as a result of different medical professionals' directly inputting the radiation therapy treatment planning data (e.g., via a platform generated/hosted by the analytics server. In other configurations, the radiation therapy treatment planning data may be at least partially retrieved from an internal or external data source.

The radiation therapy treatment planning data may include a patient identifier, patient's electronic health data records, medical images (e.g., CT scans, 4D CT Scans, MRIs, and/or X-ray images), treatment-specific data (e.g., arc information or treatment type), target organ (e.g., specification and/or location data to identify the tumor to be eradicated). Additional examples may include non-target organs, dosage-related calculations (e.g., radiation dose distribution within an anatomical region of the patient), and machine-specific recommendations (e.g., couch-gantry orientations and/or arc information).

In some configurations, the analytics server may receive (or retrieve, using the patient identifier) anatomy data associated with the patient. For instance, the analytics server may receive/retrieve data associated with the patient's anatomy, such as physical data (e.g., height, weight, and/or body mass index) and/or other health-related data (e.g., blood pressure or other data relevant to the patient receiving radiation therapy treatment). The analytics server may also receive/retrieve data associated with current and/or previous medical treatments received by the patient (e.g., data associated with the patient's previous surgeries).

The analytics server may analyze the data received/retrieved and generate additional queries accordingly. For instance, the analytics server may receive/retrieve data associated with one or more medical (or other) devices needed for the patient. The analytics server may retrieve data indicating that the patient suffers from a respiratory medical condition. As a result, the analytics server may generate and transmit a query to one or more electronic data sources to identify whether the patient uses/needs a ventilator. This information may then be used to calculate treatable sector attributes.

As will be described below, the analytics server may use patient information to identify treatable sector attributes. For instance, if the patient has a body mass index (BMI) that is higher than a predetermined threshold, and/or if the patient is utilizing a ventilator, the analytics server may limit the treatable sector and/or may not recommend a full gantry arc. This may be due to the possibility of the gantry colliding with the patient and/or the patient's ventilator. As a result, various entry angles may be eliminated from the list of possible angles that can be used for RTTP calculations.

If necessary, the analytics server may also analyze the patient's medical data records to identify the needed patient attributes. For instance, the analytics server may query a database to identify the patient's BMI. However, because many medical records are not digitalized, the analytics server may not receive the patient's BMI value using simple query techniques. As a result, the analytics server may retrieve the patient's electronic health data and may execute one or more analytical protocols (e.g., natural language processing) to identify the patient's body mass index. In another example, if the analytics server does not receive tumor data (e.g., end-points) the analytics server may execute various image recognition protocols and identify the tumor data.

The analytics server may also receive additional data from one or medical professionals. For instance, a treating oncologist may access a platform generated/hosted by the analytics server and may add, remove, or revise data associated with a particular patient, such as patient attributes, treatment attributes, tumor attributes, the primary site of treatment, tumor stage, end-point, whether the primary tumor has been extended, tumor laterality, and the like. Because tumor staging and the end-level attributes are sensitive information that affects patient treatment, this information is typically inputted by the treating oncologist. In another example, the treating oncologist may specifically indicate whether the treatment should be unilateral or bilateral.

Tumor location data may indicate the primary tumor location with respect to the patient's centerline. Therefore, the location may refer to both laterality of the tumor and its anterior/posterior position. This data may be inputted by the treating oncologist or may be analyzed using various image recognition or segmentation methods executed on the patient's medical images (that are received or retrieved by the analytics server). In some embodiments, this information can also be predicted using a machine-learning model if it is not inputted by the treating oncologist (or otherwise received by the analytics server). Another patient attribute may indicate whether and how close the tumor is to other non-diseased organs. For instance, a tumor to be eradicated may be millimeters away from another organ. This information may change field geometry, as other organs must be avoided.

In some embodiments, the analytics server may receive/retrieve, using a clinic identifier, a file comprising clinic-specific logic indicating treatment attributes of a radiation therapy machine in accordance with at least one of radiation therapy treatment planning data or patient anatomy. Each clinic implementing treatment may have its own rules and protocols regarding radiation therapy treatment. The rules may be expressed as a list of logical equations/sentences (e.g., computer-readable logic/code) referring to how the clinic usually treats patients.

At step **220,** the analytics server may execute a first computer model to identify one or more attributes of a treatable sector for the radiotherapy treatment of the patient, the first computer model configured to ingest radiation therapy treatment planning data and clinical objectives to generate the one or more attributes of the treatable sector.

Based on the patient data received and the clinical objectives, the analytics server may execute a computer model (e.g., computer model **111** in **FIG. 1****)** to identify one or more attributes of a treatable sector. The treatable sector may be calculated based on anatomical site, laterality, tumor location compared to OAR(s), and/or body outline of the patient. The computer model may include various pre-programmed logical statements that preclude certain attributes from being included within an RTTP. For instance, the computer model may determine beam entry angles that are not appropriate for certain tumors (e.g., based on tumor locations).

Based on the data received/received (e.g., step **210),** the analytics server may infer an intent for the RTTP. The intent may be based on the anatomical site and tumor laterality (e.g., left, right, bilateral, and/or no lateral). An intent for RTTP of a lung cancer patient, for example, can include information indicating that the anatomical site is lung and laterality of right. Based on the received/retrieved data, the computer model may apply one or more pre-defined algorithms to define one or more attributes of a clinically suitable treatable sector.

A treatable sector, as used herein, may refer to a range of allowable attributes of the RTTP. For instance, the treatable sector may refer to a range of allowed field (beam) entry angles in the search space (e.g., spanned by the gantry and couch rotations). In some configurations, the collision can be removed or considered as a possibility when calculating the beam entry angles (using the plan optimizer).

The computer model may use one or more pre-programmed logic and algorithms generated based on clinical practices for the corresponding clinical site, laterality, and tumor locations. For instance, the model may be generated based on previously implemented treatments and their corresponding patient data. For instance, the computer model may use an algorithm that is derived based on commonalities or other rules used for the previously-implemented treatments. The algorithms may include several variables where different variables are weighted differently.

In a non-limiting example, the algorithm may be derived in accordance with previously implemented treatments of lung cancer in conjunction with clinical objectives and/or clinic-specific rules. In some configurations, the data used to derive the algorithm (also referred to herein as the training data) may be received and monitored during previous radiation therapy treatments provided for prior patients. In another example, the training data may be a dataset that includes treatment objectives (e.g., DVH objectives), RTTPs, projected dose distribution, MLC openings, and/or control weights associated with previously treated patients.

The training data may be processed via any suitable data augmentation approach (e.g., normalization, encoding, or any combination thereof) to produce a new dataset with modified properties to improve the quality of the data. Using this data (and sometimes in conjunction with other clinical objectives or clinic-specific rules), an algorithm (or a series of algorithms) can be generated that identifies attributes for a treating sector. The algorithm may not exactly identify attributes of the RTTP itself. However, the algorithm may identify attributes of the RTTP that would not be appropriate and should not be included in the RTTP (and as a result should not be considered or evaluated by the plan optimizer). For instance, the algorithm (while not identifying an exact beam angle for the RTTP) can identify what beam angles should not be explored. This may be due to a variety of rules, clinical objectives, and/or clinic-specific requirements.

In some embodiments, the computer model may utilize machine learning algorithms and train the computer model in accordance with constraints and rules in conjunction with how previous treatments were implemented. As a result, the computer model may predict the treatable sector.

At step **230,** the analytics server may execute a second computer model to identify a radiation therapy treatment plan using the received radiation therapy treatment planning data associated with a patient and one or more attributes of the treatable sector. The analytics server may execute a second computer model (e.g., a plan optimizer, such as the plan optimizer computer model **112** depicted in **FIG. 1****).**

The second computer model may use various data, such as patient data, data received in step **210,** and the like to identify RTTP attributes for the patient's radiation therapy treatment. Conventionally, the second computer model may use the received data to iteratively calculate an RTTP within a search space. As used herein, a search space may refer to a mathematical space or range for all feasible solutions (the set of solutions among which the desired solution resides). Each point in the search space may represent one variation of a possible attribute used in the RTTP. The second computer model may iteratively identify the RTTP attributes using the search space (e.g., using different possibilities of variations for different attributes and permutations). For instance, the second model may iteratively calculate an RTTP for different values within the search space. After each iteration, the second model may evaluate the RTTP. The second model may then repeat the iteration (using a different value from the search space) and compare the RTTPs. The second model may repeat this process until the RTTP is optimized.

In a non-limiting example, the search space for beam entry angles may include ϕ = [0⁰, 360°]. Depending on model sensitivity, this search space can be divided into different segments. For instance, the search space can be divided into 360 different possibilities of 1⁰ increments or 720 different possibilities of 0.5⁰ increments. Conventionally, the second computer model would analyze each possibility within the search space. For instance, the second computer model would iteratively analyze 360 or 720 different RTTPs and select the best results. However, using the methods and systems discussed herein, the analytics server may first reduce the search space and then iteratively perform the calculations. For instance, when the treatable sector attributes indicate a ϕ = [0⁰, 85°], the analytics server may limit the search space to 85 iterations or 170 iterations (instead of 360 or 720). This reduction of the search space leads to more computational efficiency and faster results.

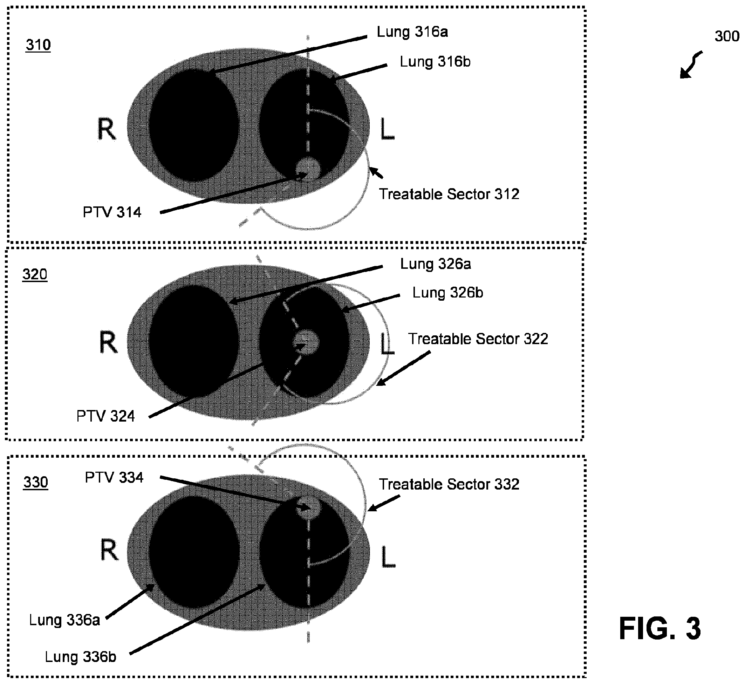

Referring now to **FIGS. 3-4****,** visual illustrations of different treatable sectors are illustrated. For instance, the example **300** illustrates three different patients' anatomies (anatomies **310, 320,** and **330).** Each anatomy depicts a tumor (PTV **314, 324,** and **334)** included within each respective patient's lungs (e.g., right lungs **316a, 326a,** and **336a** and left lungs **316b, 326b,** and **336b).** Based on the locations of the PTVs (laterality, clinical site, and tumor location), the analytics server may execute the first computer model (e.g., treatable sector computer model) to identify the treatable sectors **312, 322,** and **332.** In this example, the computer model may first assume head first supine (HFS) treatment orientation and coplanar treatment. The angle for each treatable sector may be defined as the angle between the gantry and the beam defined via a coordination system (e.g., the IEC 61217 FIXED Z-axis that is pointing upward). The algorithm may be defined as a function of tumor laterality and posteriority.

Using the training data set and rules included within the algorithm, the analytics server may calculate three different treatable sectors for the three tumors (PTVs **314, 324,** and **334).** These three treatable sectors **(312, 322,** and **332)** are examples of treatable sectors for left laterality tumors. The analytics server may calculate the treatable sector **312** as ϕ = [0°, 230°] because the PTV **314** is a posterior tumor of the lung **316b.** The analytics server may calculate the treatable sector **322** as ϕ = [330°, 220°] because the PTV **324** is located more medially in the posterior-anterior direction of the lung **326b.** Similarly, the analytics server may calculate the treatable sector **332** as ϕ = [310⁰, 180°] because the PTV **334** is an anterior tumor of the lung **336b.**

The treatable sectors depicted in **FIG. 3** visually identify a range of beam angles that are appropriate for the patient's RTTP. Therefore, the angles outside the depicted range can be eliminated from the search space used for iterative RTTP optimization calculations. While conventional methods would analyze all the applicable angles to identify the RTTP (including the beam angles), certain beam angles may not yield medically suitable results while satisfying most (if not all) mathematical requirements. For instance, to treat the PTV **314,** an RTTP may mathematically identify that the beam should enter from 100°. However, this beam angle will inevitably lead to the beam entering the patient's body from their right lung which could lead to an acceptable plan dose, but an unacceptable beam geometry due to clinical best practices of avoiding beams through the healthy lung. Therefore, mathematical formulas (without the benefit of using the medical context regarding treatable sectors) may lead to results that are mathematically acceptable but not medically acceptable. Limiting the search space used by the algorithm via the treatable sectors identified using the methods and systems discussed herein can result in fewer iterations (e.g., the model may only focus on the treatable sector angle ranges instead of 360 °), which can ultimately reduce the computational time and resources needed to optimize a patient's RTTP.

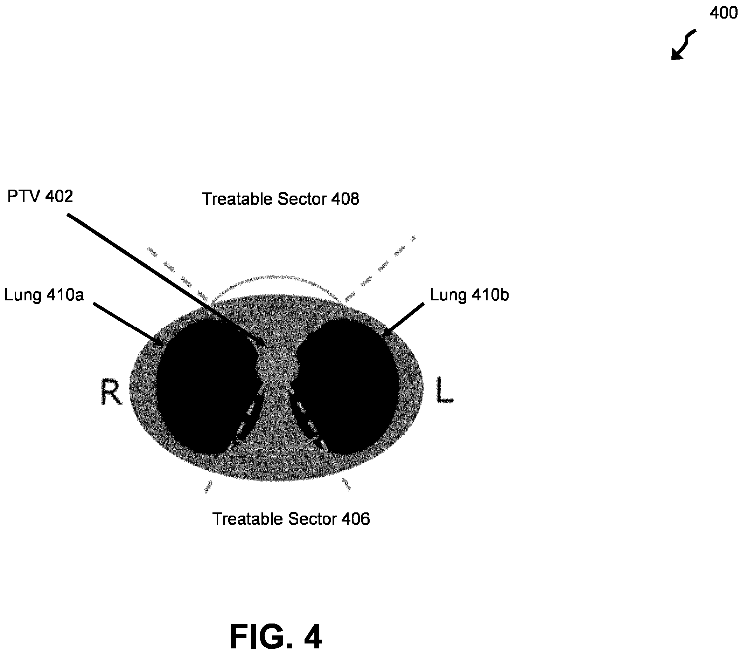

In some embodiments, the analytics server may identify two treatable sectors for a single PTV. Referring now to example **400** depicted in **FIG. 4****,** the analytics server analyzes tumor location for a PTV **402.** The example **400** illustrates two treatable sectors **406** and **408** for a patient's esophagus. In the example, the PTV is located between the patient's lungs **410a-b.** Because of the PTV's location the analytics server executes the model and identifies two ranges (front and back of the patient) illustrated as the treatable sectors **406-408:**
$ϕ = \left[{310}^{0},50°\right] and ϕ = \left[{140}^{0},220°\right]$

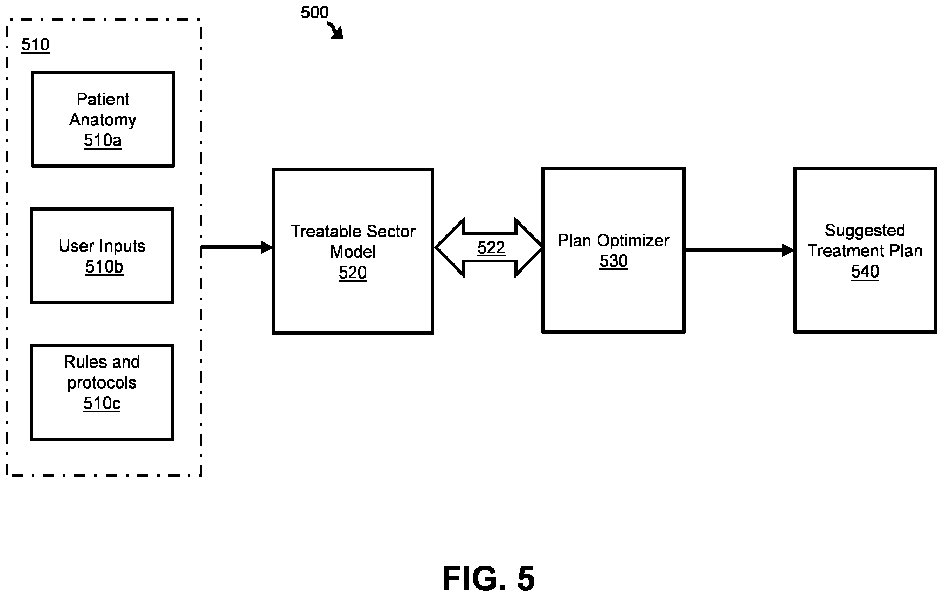

Referring now to **FIG. 5****,** a non-limiting visual example of a workflow utilizing the methods and systems described herein is illustrated. In this non-limiting example **500,** the analytics server provides treatable sector data to a plan optimizer **530** to generate a suggested RTTP that is optimized for a patient and their treatment. The analytics server may first collect patient data **510.** The patient data may include patient anatomy data **510a** (e.g., medical images, PTVs, OARs), user inputs **510b** (clinical objectives or rules received via a user interface from a treating oncologist, such as tumor data, PTV identification, and the like). Other non-limiting examples of clinical goals may include dosimetric goodness function, robustness metrics, biological effects of radiation, metrics based on linear energy transfer, and the like. The patient data **510** may also include rules **510c** for the patient's treatment (e.g., clinical/treatment objectives or criteria identified by the medical professionals or any other special treatments required by the medical professionals).

In some configurations, the analytics server may access a patient's internal/external file and retrieve/extract the needed patient data **510.** The analytics server may then execute a treatable sector model **520** to identify various attributes of a treatment sector for the patient using the patient data **510.** The results generated via the treatable sector model **520** may be ingested by the plan optimizer **530.** The plan optimizer **530** may be a treatment planning and/or monitoring software solution. The plan optimizer **530** may analyze various factors associated with the patient and the patient's treatment to generate and optimize an RTTP for the patient (e.g., field geometry, treatment modality, and radiation parameters needed to treat the patient).

One of the factors considered by the plan optimizer **530** may be the treatable sector attributes identified by the treatable sector model **520.** For instance, using the treatable sector attributes, the plan optimizer **530** may limit its search space. As a non-limiting example, when the treatable sector model **520** identifies a range of angles that are not appropriate, the plan optimizer **530** may eliminate those angles from its iterative calculation process. As a result, one or more iterations associated with the angles identified by the treatable sector model **520** will not be performed.

While the plan optimizer **530** may consider the treatable sector attributes as a factor, the plan optimizer **530** may also overwrite the results identified by the treatable sector model **520.** For instance, the RTTP generated by the plan optimizer **530** may not be dictated by the results identified by the treatable sector model **520.** The plan optimizer **530** may utilize various cost function analysis protocols where the overall plan is evaluated in light of the other (sometimes more important) factors. In some cases, other factors may be prioritized over the treatable sectors.

The plan optimizer **530** may iteratively revise the patient's RTTP where the plan optimizer **530** iteratively revises different attributes of the RTTP (e.g., field geometry). In some configurations, the plan optimizer **530** may transmit new treatment plan data back to the treatable sector model **530,** whereby the treatable sector model **530** can recalculate/re-predict data based on the revised treatment data generated by the plan optimizer (iteration **522).** The plan optimizer **530** and the treatable sector model **520** may repeat the iteration **522** until the patient's RTTP is optimized.

When the plan optimizer completes the patient's RTTP, the plan optimizer **330** may transmit the suggested treatment plan **540** to one or more electronic devices where a user (e.g., clinician) can review the suggested plan. For instance, the suggested treatment plan **540** may be displayed on a computer of a clinic where a radiation therapy technician or a treating oncologist can review the treatment plan.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc., may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The invention is defined in the following claims.

## Claims

1. A method comprising:
receiving, by a processor, radiation therapy treatment planning data associated with a radiation therapy treatment of a patient;
executing, by the processor, a first computer model to identify one or more attributes of a treatable sector for the radiation therapy treatment of the patient, the first computer model configured to ingest the radiation therapy treatment planning data to generate the one or more attributes of the treatable sector; and
executing, by the processor, a second computer model to identify a radiation therapy treatment plan for the patient using the radiation therapy treatment planning data associated with the patient, wherein the processor limits a search space used by the second computer model using the one or more attributes of the treatable sector identified by the first computer model.

2. A computer-readable medium having a set of non-transitory instructions, that when executed, cause a processor to:
receive radiation therapy treatment planning data associated with a radiation therapy treatment of a patient;
execute a first computer model to identify one or more attributes of a treatable sector for the radiation therapy treatment of the patient, the first computer model configured to ingest the radiation therapy treatment planning data to generate the one or more attributes of the treatable sector; and
execute a second computer model to identify a radiation therapy treatment plan for the patient using the radiation therapy treatment planning data associated with the patient, wherein the processor limits a search space used by the second computer model using the one or more attributes of the treatable sector identified by the first computer model.

3. The method of claim 1 or medium of claim 2, wherein the first computer model uses a machine-learning algorithm to predict the one or more attributes of the treatable sector.

4. The method or medium of claim 3, wherein the machine-learning algorithm trains the first computer model using training data comprising data associated with previously implemented treatments.

5. The method or medium of any one of claims 1 to 4, wherein the one or more attributes of the treatable sector is calculated based on tumor location.

6. The method or medium of any one of claims 1 to 5, wherein the one or more attributes of the treatable sector is calculated based on data associated with an organ at risk.

7. The method or medium of any one of claims 1 to 6, wherein the one or more attributes of the treatable sector corresponds to a range of angles associated with beam entry.

8. The method or medium of any one of claims 1 to 7, wherein the treatable sector corresponds to a plurality of ranges of beam entry angles.

9. A system comprising:
a first computer model configured to ingest radiation therapy treatment planning data to generate one or more attributes of a treatable sector for a radiation therapy treatment of a patient; and
a server in communication with the first computer model, the server configured to:
receive radiation therapy treatment planning data associated with the radiation therapy treatment of the patient;
execute the first computer model to identify the one or more attributes of the treatable sector for the radiation therapy treatment of the patient; and
execute a second computer model to identify a radiation therapy treatment plan for the patient using the radiation therapy treatment planning data associated with the patient, wherein a search space used by the second computer model is limited using the one or more attributes of the treatable sector identified by the first computer model.

10. The system of claim 9, wherein the first computer model uses a machine-learning algorithm to predict the one or more attributes of the treatable sector.

11. The system of claim 10, wherein the machine-learning algorithm trains the first computer model using training data comprising data associated with previously implemented treatments.

12. The system of claim 9, 10 or 11, wherein the one or more attributes of the treatable sector is calculated based on tumor location.

13. The system of any one of claims 9 to 12, wherein the one or more attributes of the treatable sector is calculated based on data associated with an organ at risk.

14. The system of any one of claims 9 to 13, wherein the one or more attributes of the treatable sector corresponds to a range of angles associated with beam entry.

## Patentansprüche

1. Verfahren, umfassend:
Empfangen von Strahlentherapie-Behandlungsplanungsdaten, die mit einer Strahlentherapiebehandlung eines Patienten verknüpft sind, durch einen Prozessor;
Ausführen eines ersten Computermodells durch den Prozessor, um ein oder mehrere Attribute eines behandelbaren Sektors für die Strahlentherapiebehandlung des Patienten zu ermitteln, wobei das erste Computermodell konfiguriert ist, um die Strahlentherapie-Behandlungsplanungsdaten einzulesen, um das eine oder die mehreren Attribute des behandelbaren Sektors zu erzeugen; und
Ausführen eines zweiten Computermodells durch den Prozessor, um unter Verwendung der mit dem Patienten verknüpften Strahlentherapie-Behandlungsplanungsdaten einen Strahlentherapie-Behandlungsplan für den Patienten zu ermitteln, wobei der Prozessor den durch das zweite Computermodell verwendeten Suchraum unter Verwendung des einen oder der mehreren Attribute des behandelbaren Sektors, die durch das erste Computermodell ermittelt werden, einschränkt.

2. Computerlesbares Medium, das einen Satz nichtflüchtiger Anweisungen aufweist, die bei Ausführung einen Prozessor zu Folgendem veranlassen:
Empfangen von Strahlentherapie-Behandlungsplanungsdaten, die mit einer Strahlentherapiebehandlung eines Patienten verknüpft sind;
Ausführen eines ersten Computermodells, um ein oder mehrere Attribute eines behandelbaren Sektors für die Strahlentherapiebehandlung des Patienten zu ermitteln, wobei das erste Computermodell konfiguriert ist, um die Strahlentherapie-Behandlungsplanungsdaten einzulesen, um das eine oder die mehreren Attribute des behandelbaren Sektors zu erzeugen; und
Ausführen eines zweiten Computermodells, um unter Verwendung der mit dem Patienten verknüpften Strahlentherapie-Behandlungsplanungsdaten einen Strahlentherapie-Behandlungsplan für den Patienten zu ermitteln, wobei der Prozessor den durch das zweite Computermodell verwendeten Suchraum unter Verwendung des einen oder der mehreren Attribute des behandelbaren Sektors, die durch das erste Computermodell ermittelt werden, einschränkt.

3. Verfahren nach Anspruch 1 oder Medium nach Anspruch 2, wobei das erste Computermodell einen Algorithmus für maschinelles Lernen verwendet, um das eine oder die mehreren Attribute des behandelbaren Sektors vorherzusagen.

4. Verfahren oder Medium nach Anspruch 3, wobei der Algorithmus für maschinelles Lernen das erste Computermodell unter Verwendung von Trainingsdaten trainiert, die Daten umfassen, die mit zuvor angewendeten Behandlungen verknüpft sind.

5. Verfahren oder Medium nach einem der Ansprüche 1 bis 4, wobei das eine oder die mehreren Attribute des behandelbaren Sektors basierend auf einer Position eines Tumors berechnet werden.

6. Verfahren oder Medium nach einem der Ansprüche 1 bis 5, wobei das eine oder die mehreren Attribute des behandelbaren Sektors basierend auf Daten berechnet werden, die mit einem gefährdeten Organ verknüpft sind.

7. Verfahren oder Medium nach einem der Ansprüche 1 bis 6, wobei das eine oder die mehreren Attribute des behandelbaren Sektors einem Bereich von Winkeln entsprechen, der mit einem Strahleneintritt verknüpft ist.

8. Verfahren oder Medium nach einem der Ansprüche 1 bis 7, wobei der behandelbare Sektor einer Vielzahl von Bereichen von Strahleneintrittswinkeln entspricht.

9. System, umfassend:
ein erstes Computermodell, das konfiguriert ist, um Strahlentherapie-Behandlungsplanungsdaten einzulesen, um ein oder mehrere Attribute eines behandelbaren Sektors für eine Strahlentherapiebehandlung eines Patienten zu erzeugen; und
einen Server in Kommunikation mit dem ersten Computermodell, wobei der Server zu Folgendem konfiguriert ist:
Empfangen von Strahlentherapie-Behandlungsplanungsdaten, die mit der Strahlentherapiebehandlung des Patienten verknüpft sind;
Ausführen des ersten Computermodells, um das eine oder die mehreren Attribute des behandelbaren Sektors für die Strahlentherapiebehandlung des Patienten zu ermitteln; und
Ausführen eines zweiten Computermodells, um unter Verwendung der mit dem Patienten verknüpften Strahlentherapie-Behandlungsplanungsdaten einen Strahlentherapie-Behandlungsplan für den Patienten zu ermitteln, wobei ein durch das zweite Computermodell verwendeter Suchraum unter Verwendung des einen oder der mehreren Attribute des behandelbaren Sektors, die durch das erste Computermodell ermittelt werden, eingeschränkt wird.

10. System nach Anspruch 9, wobei das erste Computermodell einen Algorithmus für maschinelles Lernen verwendet, um das eine oder die mehreren Attribute des behandelbaren Sektors vorherzusagen.

11. System nach Anspruch 10, wobei der Algorithmus für maschinelles Lernen das erste Computermodell unter Verwendung von Trainingsdaten trainiert, die Daten umfassen, die mit zuvor angewendeten Behandlungen verknüpft sind.

12. System nach Anspruch 9, 10 oder 11, wobei das eine oder die mehreren Attribute des behandelbaren Sektors basierend auf einer Position eines Tumors berechnet werden.

13. System nach einem der Ansprüche 9 bis 12, wobei das eine oder die mehreren Attribute des behandelbaren Sektors basierend auf Daten berechnet werden, die mit einem gefährdeten Organ verknüpft sind.

14. System nach einem der Ansprüche 9 bis 13, wobei das eine oder die mehreren Attribute des behandelbaren Sektors einem Bereich von Winkeln entsprechen, der mit einem Strahleneintritt verknüpft ist.

## Revendications

1. Procédé comprenant :
la réception, par un processeur, de données de planification de traitement de radiothérapie associées à un traitement de radiothérapie d'un patient ;
l'exécution, par le processeur, d'un premier modèle informatique pour identifier un ou plusieurs attributs d'un secteur traitable pour le traitement de radiothérapie du patient, le premier modèle informatique étant configuré pour ingérer les données de planification du traitement de radiothérapie afin de générer les un ou plusieurs attributs du secteur traitable ; et
l'exécution, par le processeur, d'un second modèle informatique pour identifier un plan de traitement de radiothérapie pour le patient à l'aide des données de planification du traitement de radiothérapie associées au patient, dans lequel le processeur limite un espace de recherche utilisé par le second modèle informatique à l'aide des un ou plusieurs attributs du secteur traitable identifiés par le premier modèle informatique.

2. Support lisible par ordinateur ayant un ensemble d'instructions non transitoires qui, lorsqu'elles sont exécutées, amènent un processeur à :
recevoir des données de planification de traitement de radiothérapie associées à un traitement de radiothérapie d'un patient ;
exécuter un premier modèle informatique pour identifier un ou plusieurs attributs d'un secteur traitable pour le traitement de radiothérapie du patient, le premier modèle informatique étant configuré pour ingérer les données de planification du traitement de radiothérapie afin de générer les un ou plusieurs attributs du secteur traitable ; et
exécuter un second modèle informatique pour identifier un plan de traitement de radiothérapie pour le patient à l'aide des données de planification du traitement de radiothérapie associées au patient, dans lequel le processeur limite un espace de recherche utilisé par le second modèle informatique à l'aide des un ou plusieurs attributs du secteur traitable identifiés par le premier modèle informatique.

3. Procédé selon la revendication 1 ou le support selon la revendication 2, dans lequel le premier modèle informatique utilise un algorithme d'apprentissage automatique pour prédire les un ou plusieurs attributs du secteur traitable.

4. Procédé ou le support de la revendication 3, dans lequel l'algorithme d'apprentissage automatique entraîne le premier modèle informatique à l'aide de données d'entraînement comprenant des données associées à des traitements précédemment mis en œuvre.

5. Procédé ou le support selon l'une quelconque des revendications 1 à 4, dans lequel les un ou plusieurs attributs du secteur traitable sont calculés sur la base de l'emplacement de la tumeur.

6. Procédé ou le support selon l'une quelconque des revendications 1 à 5, dans lequel les un ou plusieurs attributs du secteur traitable sont calculés sur la base de données associées à un organe à risque.

7. Procédé ou le support selon l'une quelconque des revendications 1 à 6, dans lequel les un ou plusieurs attributs du secteur traitable correspondent à une plage d'angles associés à l'entrée du faisceau.

8. Procédé ou le support selon l'une quelconque des revendications 1 à 7, dans lequel le secteur traitable correspond à une pluralité de plages d'angles d'entrée du faisceau.

9. Système comprenant :
un premier modèle informatique configuré pour ingérer des données de planification de traitement de radiothérapie afin de générer un ou plusieurs attributs d'un secteur traitable pour un traitement de radiothérapie d'un patient ; et
un serveur en communication avec le premier modèle d'ordinateur, le serveur étant configuré pour :
recevoir des données de planification de traitement de radiothérapie associées à un traitement de radiothérapie du patient ;
exécuter le premier modèle informatique pour identifier les un ou plusieurs attributs du secteur traitable pour le traitement de radiothérapie du patient ; et
exécuter un second modèle informatique pour identifier un plan de traitement de radiothérapie pour le patient à l'aide des données de planification du traitement de radiothérapie associées au patient, dans lequel un espace de recherche utilisé par le second modèle informatique est limité à l'aide des un ou plusieurs attributs du secteur traitable identifiés par le premier modèle informatique.

10. Système selon la revendication 9, dans lequel le premier modèle informatique utilise un algorithme d'apprentissage automatique pour prédire les un ou plusieurs attributs du secteur traitable.

11. Système selon la revendication 10, dans lequel l'algorithme d'apprentissage automatique entraîne le premier modèle informatique à l'aide de données d'entraînement comprenant des données associées à des traitements précédemment mis en œuvre.

12. Système selon la revendication 9, 10 ou 11, dans lequel les un ou plusieurs attributs du secteur traitable sont calculés sur la base de la localisation de la tumeur.

13. Système selon l'une quelconque des revendications 9 à 12, dans lequel les un ou plusieurs attributs du secteur traitable sont calculés sur la base de données associées à un organe à risque.

14. Système selon l'une quelconque des revendications 9 à 13, dans lequel les un ou plusieurs attributs du secteur traitable correspondent à une plage d'angles associés à l'entrée du faisceau.
